# EUROPEAN PATENT APPLICATION

(11) **EP 4 406 658 A1**
(43) Date of publication of application: **31.07.2024**
(21) Application number: 23216517.5
(22) Date of filing: 14.12.2023
(51) Int. Cl.: B04B 9/08, B04B 5/04

(54) **DIRECT DRIVE CENTRIFUGE**

(30) Priority: 28.12.2022 US 202263435672 P
(71) Applicant: Fenwal, Inc., Lake Zurich, IL 60047 (US)
(72) Inventor: MADSEN, James G., Lake Zurich, 60047 (US)
(74) Representative: Maikowski & Ninnemann Patentanwälte Partnerschaft mbB

(57) **Abstract**

A centrifuge includes a yoke driven at a rotational speed with respect to a stationary frame of the centrifuge by a drive shaft extending from a motor. A timing belt engages a stationary sun gear and a planet gear, with rotation of the yoke rotating the timing belt and the planet gear. A shaft is associated with the planet gear, with rotation of the planet gear causing the shaft and an associated first sheave to rotate within and with respect to the yoke. Another timing belt engages the first sheave, with rotation of the first sheave causing the second timing belt and a second sheave to rotate with respect to the yoke. A receptacle rotates with the second sheave to cause a fluid separation chamber to rotate with respect to the stationary frame at a speed that is double the rotational speed at which the drive shaft is driven.

## Description

### Background

### Field of the Disclosure

The present disclosure relates to centrifugal separation of fluids. More particularly, the present disclosure relates to centrifuges directly driven by timing belts and a planetary gear assembly.

### Description of Related Art

Whole blood is routinely separated into its various components, such as red blood cells, platelets, and plasma. In continuous blood processing systems, whole blood is drawn from a donor, a particular blood component or constituent is removed and collected, and the remaining blood constituents are returned to the donor. By thus removing only particular constituents, less time is needed for the donor's body to return to normal, and donations can be made at more frequent intervals than when whole blood is collected. This increases the overall supply of blood constituents, such as plasma and platelets, made available for health care.

Whole blood is typically separated into its constituents through centrifugation. This requires that the whole blood be passed through a centrifuge after it is withdrawn from, and before it is returned to, the donor. To avoid contamination, the blood is usually contained within a sealed, sterile fluid flow system during the entire centrifugation process. Typical blood processing systems thus include a permanent, reusable centrifuge assembly or "hardware" that spins and pumps the blood, and a disposable, sealed and sterile fluid processing or fluid circuit assembly that actually makes contact with the donor's blood. The centrifuge assembly engages and spins a portion of the fluid processing assembly (sometimes referred to as the separation or processing chamber) during a collection procedure. The blood, however, makes actual contact only with the disposable fluid processing assembly, which is used only once and then discarded.

To avoid the need for rotating seals, and to preserve the sterile and sealed integrity of the fluid processing assembly, continuous blood processing systems often utilize centrifuges that operate on the "one-omega, two-omega" operating principle. This principle is disclosed in detail in U.S. Patent No. 4,120,449, which is hereby incorporated herein by reference, and enables centrifuges to spin a sealed, closed system without the need for rotating seals. Blood processing systems that make use of the principle typically include a fluid processing assembly that includes a plastic bag or molded chamber that is spun in the centrifuge and that is connected to the blood donor and to a stationary portion of the centrifuge assembly through an elongated member that may be made up of one or more plastic tubes. The elongated member is commonly referred to as an "umbilicus" and is typically arranged in an upside-down question mark configuration with both of its end portions coaxially aligned with the axis of rotation of the centrifuge. The centrifuge chamber is rotated at "two-omega" RPM and the umbilicus is orbited around the centrifuge chamber at "one-omega" RPM. In other words, one end of the umbilicus is stationary, the other end rotates at a two-omega speed with the centrifuge chamber to which it is attached, and the intermediate portion or midsection of the umbilicus orbits about the chamber at a one-omega speed. The effect is that the end of the umbilicus which is opposite the bag or chamber and is connected to the donor via plastic tubing, does not twist up as the bag or chamber is spun. The sealed, sterile integrity of the fluid processing assembly is thus maintained without the need for rotating seals.

U.S. Patent No. 5,996,634, which is hereby incorporated herein by reference, discloses one such blood processing apparatus based on the "one-omega, two-omega" operating principle. In this apparatus, a disposable fluid processing assembly having an umbilicus and a processing chamber is mountable within a centrifuge assembly. One "fixed" end of the umbilicus is held rotationally stationary substantially over the axis of centrifugation. The other "free" end of the umbilicus joins the processing chamber and is free to rotate with the processing chamber around the axis of centrifugation. The mid-portion of the umbilicus is supported by a wing plate that orbits the mid-portion of the umbilicus around the axis of centrifugation at the one-omega speed. On account of having one "fixed" end and one "free" end, the umbilicus will "twist" about its own central axis as its mid-portion orbits around the processing chamber. The action of the umbilicus naturally "untwisting" itself will cause its "free" end (and, hence, the associated processing chamber) to spin at the average prescribed two-omega speed. Hence, the umbilicus itself drives the processing chamber at a two-omega average speed.

Of note is the fact that the processing chamber is not rotated at a constant or uniform speed, but rather at a two-omega average speed. This is because the untwisting action of the umbilicus tends to temporarily increase the rotational speed of the processing chamber to a level that is greater than the rotational speed of the processing chamber while the umbilicus becomes twisted about its central axis. Furthermore, on account of the umbilicus being responsible for rotating the processing chamber at the required speed, high stress levels may develop within the umbilicus, possibly leading to failures of the umbilicus. Thus, it would be advantageous to provide a system configured to avoid these possible disadvantages.

### Summary

There are several aspects of the present subject matter which may be embodied separately or together in the devices and systems described and claimed below. These aspects may be employed alone or in combination with other aspects of the subject matter described herein, and the description of these aspects together is not intended to preclude the use of these aspects separately or the claiming of such aspects separately or in different combinations as set forth in the claims appended hereto.

In one aspect, a centrifuge includes a stationary frame having a lower plate spaced from an upper support. A sun gear is fixedly secured with respect to the lower plate. The centrifuge further includes a motor and a drive shaft which extends through the lower plate from a first portion associated with the motor to a second portion adjacent to the sun gear. The motor is configured to rotate the drive shaft at a first rotational speed with respect to the upper support. A yoke positioned between the lower plate and the upper support includes a bottom platform associated to the second portion of the drive shaft and configured to be rotated with the drive shaft at the same speed. A planet gear is oriented in a common plane with the sun gear and at least partially positioned between the lower plate and the bottom platform. A yoke shaft at least partially positioned within the yoke is oriented substantially parallel to the drive shaft and extends through the bottom platform from a first end associated with the planet gear to a second end associated with a first sheave rotatably positioned within the yoke. A first timing belt engages the sun gear and the planet gear and is configured to be driven by rotation of the bottom plate to rotate the planet gear, the yoke shaft, and the first sheave. A second sheave is rotatably positioned within the yoke in a common plane with the first sheave. A second timing belt engages the first and second sheaves and is configured to be driven by rotation of the first sheave to rotate the second sheave with respect to the yoke. A receptacle is configured to rotate with the second sheave and to be connected to a fluid separation chamber so as to cause the fluid separation chamber to rotate at a second rotational speed with respect to the upper support that is double the first rotational speed.

In another aspect, a fluid separation system includes a centrifuge and a fluid flow circuit having a fluid separation chamber connected to an umbilicus. The centrifuge includes a stationary frame having a lower plate spaced from an upper support that receives a first end of the umbilicus. A sun gear is fixedly secured with respect to the lower plate. The centrifuge further includes a motor and a drive shaft which extends through the lower plate and sun gear from a first portion associated with the motor to a second portion adjacent to the sun gear. The motor is configured to rotate the drive shaft at a first rotational speed with respect to the upper support. A yoke positioned between the lower plate and the upper support includes a bottom platform associated to the second portion of the drive shaft and configured to be rotated with the drive shaft at the same speed. A planet gear is oriented in a common plane with the sun gear and at least partially positioned between the lower plate and the bottom platform. A yoke shaft at least partially positioned within the yoke is oriented substantially parallel to the drive shaft and extends through the bottom platform from a first end associated with the planet gear to a second end associated with a first sheave rotatably positioned within the yoke. A first timing belt engages the sun gear and the planet gear and is configured to be driven by rotation of the bottom platform to rotate the planet gear, the yoke shaft, and the first sheave. A second sheave is rotatably positioned within the yoke in a common plane with the first sheave. A second timing belt engages the first and second sheaves and is configured to be driven by rotation of the first sheave to rotate the second sheave with respect to the yoke. A receptacle connected to the fluid separation chamber is configured to rotate with the second sheave so as to cause the fluid separation chamber to rotate at a second rotational speed with respect to the upper support that is double the first rotational speed.

In yet another aspect, a method of centrifugally separating a fluid includes mounting a first end of an umbilicus to an upper support of a stationary frame of a centrifuge and mounting a fluid separation chamber connected to the umbilicus to a receptacle of the centrifuge. A motor of the centrifuge is operated to rotate a drive shaft of the centrifuge at a first rotational speed with respect to the upper support so as to cause the receptacle, the fluid separation chamber, and a fluid within the fluid separation chamber to be rotated at a second rotational speed with respect to the upper support that is double the first rotational speed. A bottom platform of a yoke of the centrifuge is associated with the drive shaft such that rotation of the drive shaft causes rotation of the yoke, with a first timing belt engaging a sun gear fixedly secured with respect to a lower plate of the stationary frame and a planet gear so as to transmit rotation of the bottom platform to the planet gear. A yoke shaft at least partially positioned within the yoke is associated with the planet gear and rotates with the planet gear, while a first sheave associated with the yoke shaft and positioned within the yoke rotates with the yoke shaft and the planet gear. A second timing belt engages the first sheave and a second sheave positioned within the yoke so as to transmit rotation of the first sheave to the second sheave, with the receptacle being configured to rotate with the second sheave.

### Brief Description of the Drawings

Fig. 1 is a front elevational view of a centrifuge according to an aspect of the present disclosure, with a fluid separation chamber of a fluid flow circuit mounted thereto;
Fig. 2 is a front elevational view of the centrifuge of Fig. 1, with the fluid separation chamber being omitted;
Fig. 3 is a cross-sectional view of the centrifuge of Fig. 2;
Fig. 4 is a perspective view of a yoke and drive assembly of the centrifuge of Fig. 2;
Fig. 5 is a cross-sectional view of the yoke and drive assembly of Fig. 4;
Fig. 6 is a perspective view of the centrifuge of Fig. 2, with portions of the yoke thereof omitted for illustrative purposes;
Fig. 7 is perspective view of the centrifuge of Fig. 2, with the yoke thereof omitted for illustrative purposes;
Fig. 8 is a cross-sectional view of the centrifuge of Fig. 7, with portions of a frame thereof omitted for illustrative purposes;
Fig. 9 is a top plan view of the centrifuge of Fig. 8; and
Fig. 10 is a bottom plan view of the centrifuge of Fig. 2, with portions of the frame and drive assembly thereof omitted for illustrative purposes.

### Description of the Illustrated Embodiments

The embodiments disclosed herein are for the purpose of providing a description of the present subject matter, and it is understood that the subject matter may be embodied in various other forms and combinations not shown in detail. Therefore, specific embodiments and features disclosed herein are not to be interpreted as limiting the subject matter as defined in the accompanying claims.

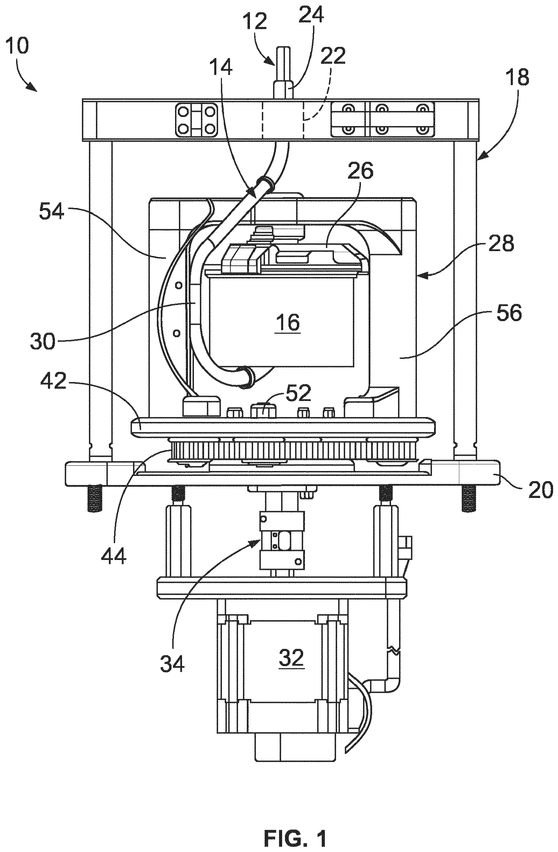

Fluid separation systems according to the present disclosure include a separation device, which may be variously configured without departing from the scope of the present disclosure. Figs. 1-10 show a centrifuge 10 of an exemplary durable separation device that may be employed in fluid separation systems according to the present disclosure. The separation device can be used for processing various fluids, but is particularly well-suited for processing whole blood and other suspensions of biological cellular materials. While fluid separation principles will be described herein with reference to one particular system, it should be understood that these principles may be employed with other fluid separation systems and separation devices without departing from the scope of the present disclosure.

Fig. 1 also illustrates components of a disposable flow circuit 12 that may be used in combination with the separation device to provide a fluid separation system. The flow circuit 12 includes a variety of tubing and a number of components, only some of which will be described herein in greater detail. It should be understood that Fig. 1 illustrates only one example of a flow circuit which may be used in combination with a separation device according to the present disclosure and that differently configured flow circuits may also be employed.

The illustrated flow circuit 12 includes, among other things, an umbilicus 14 connected to a fluid separation chamber 16. The fluid separation chamber 16 and umbilicus 14 may be variously configured without departing from the scope of the present disclosure. However, in one exemplary embodiment, the fluid separation chamber 16 may be configured as described in U.S. Patent Application Publication No. 2022/0314237 A1 (which is hereby incorporated herein by reference) and the umbilicus 14 may be configured as described in U.S. Patent No. 5,996,634.

The umbilicus 14 is configured to direct a fluid to be separated into the fluid separation chamber 16 and then direct the separated fluid components out of the fluid separation chamber 16. Typically, an umbilicus will include one lumen for conveying a fluid (e.g., blood) into a fluid separation chamber and multiple lumens for conveying separated fluid components (e.g., packed red blood cells and platelet-rich plasma) out of the fluid separation chamber. As for the fluid separation chamber 16, it will typically include an inlet leading into a generally annular channel, with multiple outlets (corresponding to the number of outlet lumens of the umbilicus) leading out of the channel. At least one of the outlets is associated with a radially inner "low-G" wall of the channel for conveying a relatively low-density fluid component (e.g., platelet-poor plasma) out of the channel, while at least one of the outlets is associated with a radially outer "high-G" wall of the channel for conveying a relatively high-density fluid component (e.g., packed red blood cells) out of the channel.

The centrifuge 10 includes a stationary frame 18 having a lower plate 20 spaced from an upper support 22. It should be understood that the terms "lower" and "upper" are simply used to refer to the orientation of the centrifuge 10 shown in Figs. 1-10, rather than requiring a particular centrifuge configuration.

A first end 24 of the umbilicus 14 is secured to the upper support or holder 22, with the upper support 22 preventing rotation of the first end 24 of the umbilicus 14. The fluid separation chamber 16 is mounted or secured to a receptacle 26 of the centrifuge 10 that is coaxial with the upper support 22. A yoke 28 of the centrifuge 10 includes a central support 30 configured to engage a midsection of the umbilicus 14, allowing the midsection of the umbilicus 14 to rotate with respect to the central support 30. As will be described in greater detail herein, the yoke 28 is rotated at a first rotational speed (referred to herein as the "one-omega" speed) with respect to the upper support 22 (and the remainder of the frame 18), while the receptacle 26 and fluid separation chamber 16 rotate at a second rotational speed (referred to herein as the "two-omega" speed) that is double the first rotational speed.

In the illustrated embodiment, the receptacle 26 is configured to grip onto an upper end of the fluid separation chamber 16, but it should be understood that the receptacle 26 may be differently configured, depending on the configuration of the associated fluid separation chamber 16. For example, the fluid separation chamber 16 of Fig. 1 is formed of a generally rigid material having a defined shape, such that it is sufficient for a relatively small and/or simple receptacle 26 to be provided. In other embodiments, the fluid separation chamber may be configured as a bag or belt formed of a generally flexible material that requires the receptacle to dictate the shape of the fluid separation chamber during centrifugation. In this case, the receptacle may include a generally cylindrical spool surrounded by a bowl, with a substantially annular groove defined therebetween. The fluid separation chamber is mounted to the receptacle within this groove to give the fluid separation chamber a substantially annular configuration.

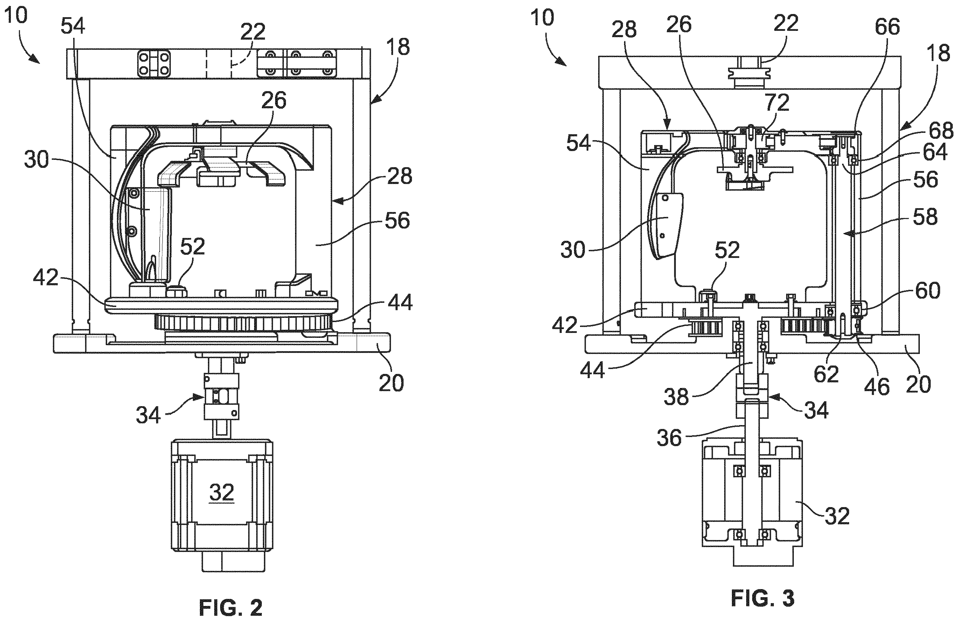

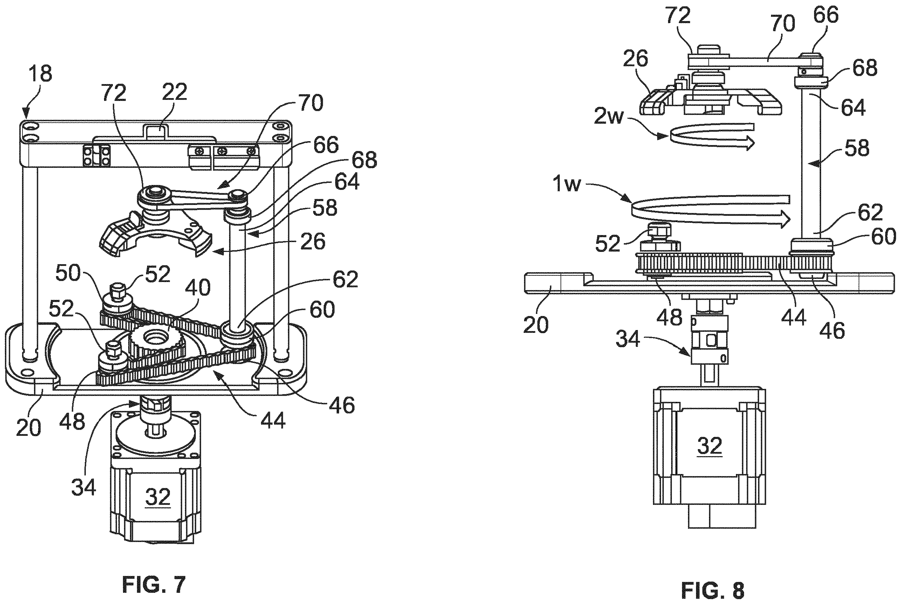

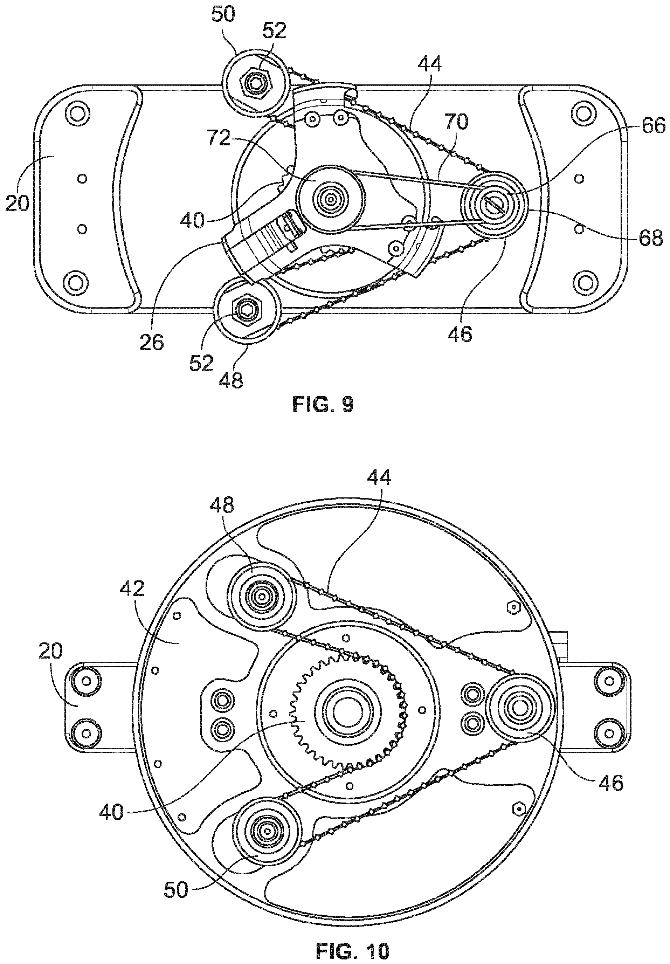

Turning now to the configuration of the centrifuge 10, it includes a motor 32 secured to the lower plate 20 of the frame 18. A sun gear 40 is fixedly secured with respect to the lower plate 20, which may include the sun gear 40 being machined into the lower plate 20 or otherwise affixed to the lower plate 20 (e.g., via one or more welds or mechanical fasteners). A drive shaft 34 extends through the lower plate 20 and the sun gear 40, from a first portion 36 associated with the motor 32 to a second portion 38 (Fig. 3) adjacent to the sun gear 40 (Figs. 7 and 10). The drive shaft 34 and sun gear 40 are substantially coaxial with the upper support 22, defining a rotational axis of the yoke 28 (as will be described in greater detail).

The motor 32 is controlled by a controller of the separation device to rotate the drive shaft 34 at a desired "one-omega" speed. The selected "one-omega" speed is based on the rate at which the fluid separation chamber 16 is to be rotated with respect to the upper support 22. More particularly, the "one-omega" speed is half the "two-omega" speed at which the fluid separation chamber 16 rotates with respect to the upper support 22 so, if it desired for the fluid separation chamber 16 to rotate at 4,500 RPM with respect to the upper support 22, the controller will command the motor 32 to operate so as to rotate the drive shaft 34 at a "one-omega" speed of 2,250 RPM. It should be understood that the rate at which the motor 32 rotates the drive shaft 34 may vary during the course of a fluid separation procedure, with the resulting rotational speed of the fluid separation chamber 16 similarly varying (but always being twice the rotational speed of the drive shaft 34 with respect to the upper support 22).

The second portion 38 of the drive shaft 34 is secured with respect to a bottom platform 42 of the yoke 28, such that rotation of the drive shaft 34 will cause the bottom platform 42 (and the remainder of the yoke 28) to rotate at the same speed (i.e., the "one-omega" speed). In the illustrated embodiment, the bottom platform 42 of the yoke 28 is substantially circular, but it should be understood that the bottom platform 42 may be differently shaped without departing from the scope of the present disclosure.

As can be seen in Fig. 1, the lower plate 20 of the frame 18 is spaced from the bottom platform 42 of the yoke 28, with at least a portion of the sun gear 40 and an associated timing belt 44 positioned therebetween. In addition to engaging the stationary sun gear 40, the timing belt 44 also engages a planet gear 46 that is oriented in the same plane as the sun gear 40. The relative positions of the sun gear 40 and the planet gear 46 in the planetary gear layout can be best seen in Figs. 9 and 10, with two additional planet gears 48 and 50 also being shown in engagement with the timing belt 44. Fig. 10 shows the planet gears 46, 48, and 50 as being substantially identical and oriented in a triangle with the stationary sun gear 40 adjacent to a center of the triangle, but it should be understood that the individual planet gears 46, 48, and 50 may be differently configured and arranged without departing from the scope of the present disclosure. However, the illustrated orientation may be advantageous to ensure that the timing belt 44 is securely engaged with the sun gear 40 and first planet gear 46 so that rotation of the bottom platform 42 of the yoke 28 is properly transmitted to the first planet gear 46 (as will be described in greater detail).

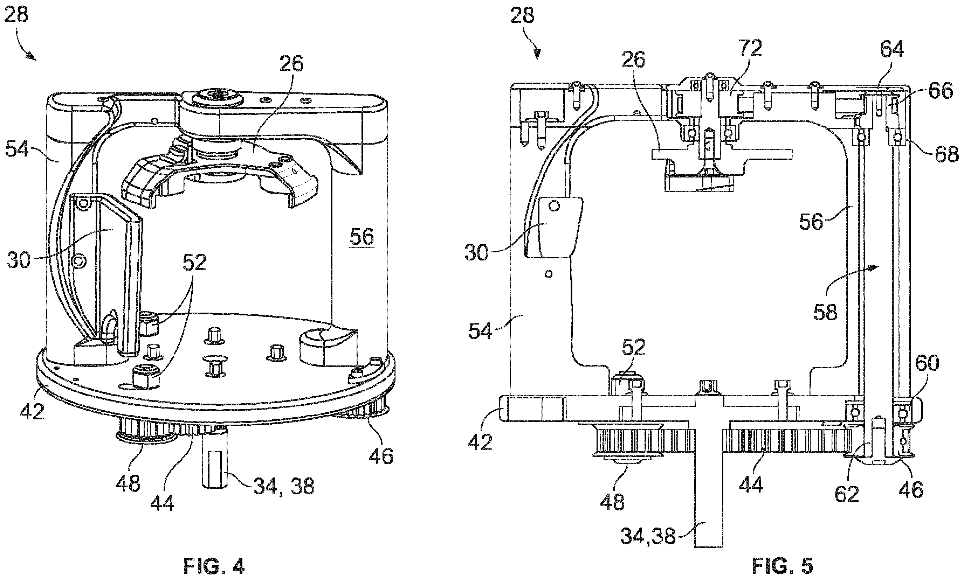

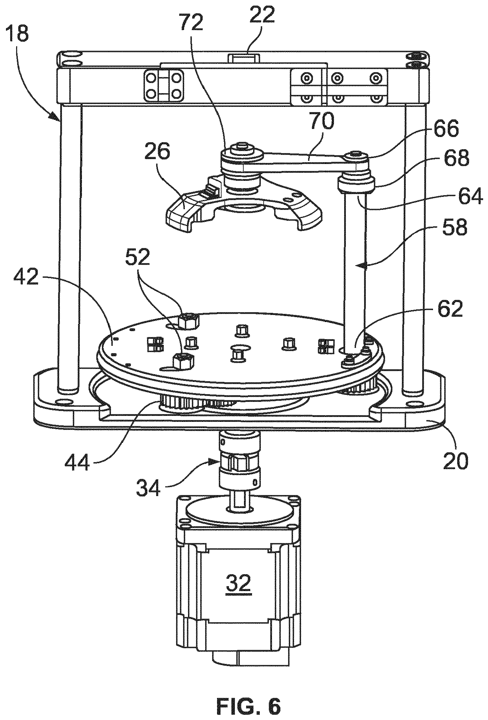

The planet gears 46, 48, and 50 are spaced away from the lower plate 20 of the frame 18, while being rotatably associated to the bottom platform 42 of the yoke 28. For example, Figs. 4 and 6 show mechanical fasteners 52 used to join the second and third planet gears 48 and 50 to the bottom platform 42. As for the first planet gear 46, it is used to transmit rotation to the receptacle 26 (as will be described in greater detail herein), so it may be differently associated to the yoke 28, such as by a bearing 60. Indeed, the planet gears 46, 48, and 50 may be associated to the yoke 28 according to any suitable approach that allows them to both rotate along with the yoke 28 at the "one-omega" speed dictated by operation of the motor 32, with each planet gear 46, 48, and 50 also rotating about its own central axis with respect to the bottom platform 42 and the remainder of the yoke 28 (as will be described below).

In an exemplary embodiment (which can be seen in Figs. 3 and 5), the yoke 28 includes two arms 54 and 56, with the central support 30 being associated with the first arm 54. On account of the central support 30 of the yoke 28 engaging the midsection of the umbilicus 14, the midsection of the umbilicus 14 will orbit about the rotational axis of the yoke 28 at the same "one-omega" rotational speed, while the midsection of the umbilicus 14 is allowed to rotate about its own central axis with respect to the central support 30. As for the second arm 56 of the yoke 28, a yoke shaft 58 extending from the first planet gear 46 is rotatably received therewithin (and oriented substantially parallel to the drive shaft 34). In this illustrated embodiment, the yoke shaft 58 extends through the bearing 60 that is associated with the bottom platform 42 of the yoke 28. As will be described in greater detail, the yoke shaft 58 cooperates with the first planet gear 46 to transmit rotation to the receptacle 26.

As noted above, the first timing belt 44 engages the planet gears 46, 48, and 50, such that rotation of the planet gears 46, 48, and 50 with the bottom platform 42 of the yoke 28 also causes the first timing belt 44 to rotate about the sun gear 40. As the sun gear 40 is stationary (while each planet gear 46, 48, and 50 is free to rotate about its own central axis with respect to the bottom plate 42 of the yoke 28), rotation of the first timing belt 44 about the sun gear 40 will cause each planet gear 46, 48, and 50 to rotate with respect to the bottom platform 42. The exact rate at which each planet gear 46, 48, and 50 rotates with respect to the bottom platform 42 will depend upon the configurations of the various gears 40, 46, 48, and 50 (i.e., the relative diameters and number of teeth of the planet gears 46, 48, and 50 with respect to the diameter and number of teeth of the sun gear 40) and may vary without departing from the scope of the present disclosure. However, as it is desirable for the fluid separation chamber 16 and receptacle 26 to rotate at a rate that is double the rate at which the drive shaft 34 and yoke 28 are rotated (with respect to the upper support 22), it may be advantageous for the planet gears 46, 48, and 50 (or at least the first planet gear 46) to have a smaller diameter and fewer teeth than the sun gear 40, though the relative sizes of the gears may vary without departing from the scope of the present disclosure.

The yoke shaft 58 extends from a first end 62 associated with the first planet gear 46 to a second end 64 associated with a sheave 66 that is also rotatably positioned within the yoke 28. As shown in Figs. 5 and 7, another bearing 68 may be associated with the second end 64 of the yoke shaft 58, with the two bearings 60 and 68 retaining the yoke shaft 58 in the proper orientation within the arm 56 of the yoke 28, while allowing the yoke shaft 58 to rotate along with the associated planet gear 46.

The sheave 66 is engaged by a second timing belt 70, which also engages a second sheave 72 (positioned in a common plane with the first sheave 66), as shown in Figs. 6-9. Figs. 6-9 omit the yoke 28 to better illustrate the drivetrain between the motor 32 and the receptacle 26, which is configured to rotate with the second sheave 72. However, it will be seen in Figs. 3 and 5 that the second sheave 72 is rotatably positioned within the yoke 28, similar to the yoke shaft 58 and the first sheave 66. On the other hand, the receptacle 26 is positioned outside of the yoke 28 so as to be able to be connected to the fluid separation chamber 16 of the fluid flow circuit 12. The receptacle 26 and second sheave 72 are substantially coaxial with the drive shaft 34, the sun gear 40, and the upper support 22, along the rotational axis of the yoke 28.

Regardless of the particular speed at which the first planet gear 46 rotates, it causes the yoke shaft 58 and the first sheave 66 to rotate at the same rate, as a unitary structure. The second timing belt 70 transfers the rotation of the first sheave 66 to the second sheave 72, though the two sheaves 66 and 72 will not necessarily rotate at the same speed. Instead, the speed at which the second sheave 72 (and associated receptacle 26) rotates will depend upon the relative diameters and number of teeth of the two sheaves 66 and 72. As described above, it is desirable for the second sheave 72 and associated receptacle 26 to rotate at a rate that is twice the "one-omega" rotational speed of the drive shaft 34, sun gear 40, and yoke 28 with respect to the upper support 22. If the second sheave 72 and receptacle 26 are to be rotated at a greater rate than the first sheave 66 (i.e., when the first sheave 66 is rotating about its central axis at a rate that is less than the "two-omega" speed), the second sheave 72 should have a diameter that is smaller than the diameter of the first sheave 66. On the other hand, if the second sheave 72 and receptacle 26 are to be rotated at a lower rate than the first sheave 66 (i.e., when the first sheave 66 is rotating about its central axis at a rate that is greater than the "two-omega" speed), the second sheave 72 should have a diameter that is larger than the diameter of the first sheave 66. In yet another embodiment, it may be desirable for the second sheave 72 to rotate at the same rate as the first sheave 66 (i.e., when the first sheave 66 is rotating at the desired "two-omega" speed), in which case the two sheaves 66 and 72 may have the same configuration. Thus, it should be understood that the configurations of the individual gears 40 and 46 and sheaves 66 and 72 may vary without departing from the scope of the present disclosure, though the configuration of each will depend upon the configurations of the others so as to ensure that the receptacle 26 and fluid separation chamber 16 are rotated at the proper "two-omega" speed.

On account of the system not relying upon the umbilicus 14 to rotate the fluid separation chamber 16, the umbilicus 14 is not subject to the same high stress levels that may develop within the umbilicus of an umbilicus-driven system. As the umbilicus 14 is subject to lower levels of stress, it may be differently configured than a conventional umbilicus, which may allow for a simpler, less expensive umbilicus to be used in systems according to the present disclosure.

It should be understood that the illustrated embodiment is merely exemplary and that the principles described herein may be practiced with differently configured system components. For example, the first timing belt 44 is illustrated as having teeth on its opposing surfaces that mesh with teeth of the gears 40, 46, 48, and 50. In an alternative embodiment, the gears 40, 46, 48, and 50 may be replaced by sheaves omitting teeth, with the timing belt 44 similarly omitting teeth and being configured as a simplified belt or loop of material (e.g., rubber or another elastomeric material). Similarly, while the second timing belt 70 is illustrated as a simplified belt or loop of material that engages the two sheaves 66 and 72, it is contemplated that the two sheaves 66 and 72 could be replaced with gears and for the second timing belt 70 to be provided with teeth that mesh with the teeth of such gears. Other modifications to the illustrated system components may also be employed without departing from the scope of the present disclosure.

### Aspects

Aspect 1. A centrifuge, comprising: a stationary frame including a lower plate spaced from an upper support; a sun gear fixedly secured with respect to the lower plate; a motor; a drive shaft extending through the lower plate from a first portion associated with the motor to a second portion adjacent to the sun gear, wherein the motor is configured to rotate the drive shaft at a first rotational speed with respect to said upper support; a yoke positioned between the lower plate and the upper support and including a bottom platform associated to the second portion of the drive shaft and configured to be rotated with the drive shaft at said first rotational speed; a planet gear oriented in a common plane with the sun gear and at least partially positioned between the lower plate and the bottom platform; a yoke shaft at least partially positioned within the yoke, oriented substantially parallel to the drive shaft, and extending through the bottom platform from a first end associated with the planet gear to a second end associated with a first sheave rotatably positioned within the yoke; a first timing belt engaging the sun gear and the planet gear and configured to be driven by rotation of the bottom platform to rotate the planet gear, the yoke shaft, and the first sheave; a second sheave rotatably positioned within the yoke in a common plane with the first sheave; a second timing belt engaging the first and second sheaves and configured to be driven by rotation of the first sheave to rotate the second sheave with respect to the yoke; and a receptacle configured to rotate with the second sheave and to be connected to a fluid separation chamber so as to cause the fluid separation chamber to rotate at a second rotational speed with respect to said upper support that is double the first rotational speed.

Aspect 2. The centrifuge of Aspect 1, wherein the upper support is configured to receive a first end of an umbilicus extending from the fluid separation chamber so as to prevent rotation of said first end, and the yoke includes a central support configured to engage a midsection of the umbilicus so as to cause the midsection of the umbilicus to rotate with the yoke.

Aspect 3. The centrifuge of Aspect 2, wherein the yoke includes first and second arms, the central support is associated with the first arm, and the yoke shaft is rotatably received within the second arm.

Aspect 4. The centrifuge of any one of the preceding Aspects, further comprising second and third planet gears each engaging the first timing belt and configured to be rotated by the first timing belt.

Aspect 5. The centrifuge of any one of the preceding Aspects, wherein the second sheave is substantially coaxial with the drive shaft, the sun gear, and the upper support.

Aspect 6. A fluid separation system, comprising: a fluid flow circuit including a fluid separation chamber connected to an umbilicus; and a centrifuge comprising a stationary frame including a lower plate spaced from an upper support receiving a first end of the umbilicus, a sun gear fixedly secured with respect to the lower plate, a motor, a drive shaft extending through the lower plate and sun gear from a first portion associated with the motor to a second portion adjacent to the sun gear, wherein the motor is configured to rotate the drive shaft at a first rotational speed with respect to said upper support, a yoke positioned between the lower plate and the upper support and including a bottom platform associated to the second portion of the drive shaft and configured to be rotated with the drive shaft at said first rotational speed, a planet gear oriented in a common plane with the sun gear and at least partially positioned between the lower plate and the bottom platform, a yoke shaft at least partially positioned within the yoke, oriented substantially parallel to the drive shaft, and extending through the bottom platform from a first end associated with the planet gear to a second end associated with a first sheave rotatably positioned within the yoke, a first timing belt engaging the sun gear and the planet gear and configured to be driven by rotation of the bottom platform to rotate the planet gear, the yoke shaft, and the first sheave, a second sheave rotatably positioned within the yoke in a common plane with the first sheave, a second timing belt engaging the first and second sheaves and configured to be driven by rotation of the first sheave to rotate the second sheave with respect to the yoke, and a receptacle configured to rotate with the second sheave and connected to the fluid separation chamber so as to cause the fluid separation chamber to rotate at a second rotational speed with respect to said upper support that is double the first rotational speed.

Aspect 7. The fluid separation system of Aspect 6, wherein the upper support is configured to prevent rotation of said first end of the umbilicus, and the yoke includes a central support configured to engage a midsection of the umbilicus so as to cause the midsection of the umbilicus to rotate with the yoke.

Aspect 8. The fluid separation system of Aspect 7, wherein the yoke includes first and second arms, the central support is associated with the first arm, and the yoke shaft is rotatably received within the second arm.

Aspect 9. The fluid separation system of any one of Aspects 6-8, further comprising second and third planet gears each engaging the first timing belt and configured to be rotated by the first timing belt.

Aspect 10. The fluid separation system of any one of Aspects 6-9, wherein the second sheave is substantially coaxial with the drive shaft, the sun gear, and the upper support.

Aspect 11. A method of centrifugally separating a fluid, comprising: mounting a first end of an umbilicus to an upper support of a stationary frame of a centrifuge; mounting a fluid separation chamber connected to the umbilicus to a receptacle of the centrifuge; and operating a motor of the centrifuge to rotate a drive shaft of the centrifuge at a first rotational speed with respect to said upper support so as to cause the receptacle, the fluid separation chamber, and a fluid within the fluid separation chamber to be rotated at a second rotational speed with respect to said upper support that is double the first rotational speed, wherein a bottom platform of a yoke of the centrifuge is associated with the drive shaft such that rotation of the drive shaft causes rotation of the yoke, a first timing belt engages a sun gear fixedly secured with respect to a lower plate of the stationary frame and a planet gear so as to transmit rotation of the bottom platform to the planet gear, a yoke shaft at least partially positioned within the yoke and associated with the planet gear rotates with the planet gear, a first sheave associated with the yoke shaft and positioned within the yoke rotates with the yoke shaft and the planet gear, a second timing belt engages the first sheave and a second sheave positioned within the yoke so as to transmit rotation of the first sheave to the second sheave, and the receptacle is configured to rotate with the second sheave.

Aspect 12. The method of Aspect 11, wherein the upper support is configured to prevent rotation of said first end of the umbilicus, and the yoke includes a central support configured to engage a midsection of the umbilicus so as to cause the midsection of the umbilicus to rotate with the yoke.

Aspect 13. The method of Aspect 12, wherein the yoke includes first and second arms, the central support is associated with the first arm, and the yoke shaft is rotatably received within the second arm.

Aspect 14. The method of any one of Aspects 11-13, further comprising second and third planet gears each engaging the first timing belt and configured to be rotated by the first timing belt.

Aspect 15. The method of any one of Aspects 11-14, wherein the second sheave is substantially coaxial with the drive shaft, the sun gear, and the upper support.

It will be understood that the embodiments and examples described above are illustrative of some of the applications of the principles of the present subject matter. Numerous modifications may be made by those skilled in the art without departing from the spirit and scope of the claimed subject matter, including those combinations of features that are individually disclosed or claimed herein. For these reasons, the scope hereof is not limited to the above description but is as set forth in the following claims, and it is understood that claims may be directed to the features hereof, including as combinations of features that are individually disclosed or claimed herein.

## Claims

1. A centrifuge (10), comprising:
a stationary frame (18) including a lower plate (20) spaced from an upper support (22);
a sun gear (40) fixedly secured with respect to the lower plate (20);
a motor (32);
a drive shaft (34) extending through the lower plate (20) and sun gear (40) from a first portion (36) associated with the motor (32) to a second portion (38) adjacent to the sun gear (40), wherein the motor (32) is configured to rotate the drive shaft (34) at a first rotational speed with respect to said upper support (22);
a yoke (28) positioned between the lower plate (20) and the upper support (22) and including a bottom platform (42) associated to the second portion (38) of the drive shaft (34) and configured to be rotated with the drive shaft (34) at said first rotational speed;
a planet gear (46) oriented in a common plane with the sun gear (40) and at least partially positioned between the lower plate (20) and the bottom platform (42);
a yoke shaft (58) at least partially positioned within the yoke (28), oriented substantially parallel to the drive shaft (34), and extending through the bottom platform (42) from a first end (62) associated with the planet gear (46) to a second end (64) associated with a first sheave (66) rotatably positioned within the yoke (28);
a first timing belt (44) engaging the sun gear (40) and the planet gear (46) and configured to be driven by rotation of the bottom platform (42) to rotate the planet gear (46), the yoke shaft (58), and the first sheave (66);
a second sheave (72) rotatably positioned within the yoke (28) in a common plane with the first sheave (66);
a second timing belt (70) engaging the first and second sheaves (66, 72) and configured to be driven by rotation of the first sheave (66) to rotate the second sheave (72) with respect to the yoke (28); and
a receptacle (26) configured to rotate with the second sheave (72) and to be connected to a fluid separation chamber (16) so as to cause the fluid separation chamber (16) to rotate at a second rotational speed with respect to said upper support (22) that is double the first rotational speed.

2. The centrifuge (10) of claim 1, wherein
the upper support (22) is configured to receive a first end (24) of an umbilicus (14) extending from the fluid separation chamber (16) so as to prevent rotation of said first end (24), and
the yoke (28) includes a central support (30) configured to engage a midsection of the umbilicus (14) so as to cause the midsection of the umbilicus (14) to rotate with the yoke (28).

3. The centrifuge (10) of claim 2, wherein
the yoke (28) includes first and second arms (54, 56),
the central support (30) is associated with the first arm (54), and
the yoke shaft (58) is rotatably received within the second arm (56).

4. The centrifuge (10) of any one of the preceding claims, further comprising second and third planet gears (48, 50) each engaging the first timing belt (44) and configured to be rotated by the first timing belt (44).

5. The centrifuge (10) of any one of the preceding claims, wherein the second sheave (72) is substantially coaxial with the drive shaft (34), the sun gear (40), and the upper support (22).

6. A fluid separation system, comprising:
a fluid flow circuit (12) including a fluid separation chamber (16) connected to an umbilicus (14); and
the centrifuge (10) of any one of the preceding claims, wherein
the stationary frame (18) receives a first end (24) of the umbilicus (14), and
the receptacle (26) is connected to the fluid separation chamber (16).

7. A method of centrifugally separating a fluid, comprising:
mounting a first end (24) of an umbilicus (14) to an upper support (22) of a stationary frame (18) of a centrifuge (10);
mounting a fluid separation chamber (16) connected to the umbilicus (14) to a receptacle (26) of the centrifuge (10); and
operating a motor (32) of the centrifuge (10) to rotate a drive shaft (34) of the centrifuge (10) at a first rotational speed with respect to said upper support (22) so as to cause the receptacle (26), the fluid separation chamber (16), and a fluid within the fluid separation chamber (16) to be rotated at a second rotational speed with respect to said upper support (22) that is double the first rotational speed, wherein
a bottom platform (42) of a yoke (28) of the centrifuge (10) is associated with the drive shaft (34) such that rotation of the drive shaft (34) causes rotation of the yoke (28),
a first timing belt (44) engages a sun gear (40) fixedly secured with respect to a lower plate (20) of the stationary frame (18) and a planet gear (46) so as to transmit rotation of the bottom platform (42) to the planet gear (46),
a yoke shaft (58) at least partially positioned within the yoke (28) and associated with the planet gear (46) rotates with the planet gear (46),
a first sheave (66) associated with the yoke shaft (58) and positioned within the yoke (28) rotates with the yoke shaft (58) and the planet gear (46),
a second timing belt (70) engages the first sheave (66) and a second sheave (72) positioned within the yoke (28) so as to transmit rotation of the first sheave (66) to the second sheave (72), and
the receptacle (26) is configured to rotate with the second sheave (72).

8. The method of claim 7, wherein
the upper support (22) is configured to prevent rotation of said first end (24) of the umbilicus (14), and
the yoke (28) includes a central support (30) configured to engage a midsection of the umbilicus (14) so as to cause the midsection of the umbilicus (14) to rotate with the yoke (28).

9. The method of claim 8, wherein
the yoke (28) includes first and second arms (54, 56),
the central support (30) is associated with the first arm (54), and
the yoke shaft (58) is rotatably received within the second arm (56).

10. The method of any one of claims 7-9, further comprising second and third planet gears (48, 50) each engaging the first timing belt (44) and configured to be rotated by the first timing belt (44).

11. The method of any one of claims 7-10, wherein the second sheave (72) is substantially coaxial with the drive shaft (34), the sun gear (40), and the upper support (22).
